# EUROPEAN PATENT SPECIFICATION

(11) **EP 2 654 746 B1**
(45) Date of publication and mention of the grant of the patent: **10.08.2016**
(21) Application number: 11811111.1
(22) Date of filing: 20.12.2011
(51) Int. Cl.: A61K 31/343, A61K 33/24, A61K 33/34, A61K 9/06, A61K 9/12, A61K 9/00, A61P 17/10, A61P 31/04, A61L 101/02, A61L 101/26, A61L 101/44

(54) **ANTIBACTERIAL OR ANTI-ACNE FORMULATIONS CONTAINING USNIC ACID OR AN USNATE AND A METAL SALT**
ANTIBAKTERIELLE FORMULIERUNGEN BZW. FORMULIERUNGEN GEGEN AKNE MIT USNINSÄURE ODER EINEM USNAT- UND EINEM METALLSALZ
FORMULATIONS ANTIBACTÉRIENNES OU ANTI-ACNÉIQUES CONTENANT DE L'ACIDE USNIQUE OU UN USNATE ET UN SEL DE MÉTAL

(30) Priority: 22.12.2010 GB 201021745
(43) Date of publication of application: 30.10.2013
(73) Proprietor: Innovenn Limited, Dalkey Dublin (IE)
(72) Inventor: EADY, Elizabeth Anne, Yorkshire LS22 7TW (GB); FITZGERALD, Daniel James, Yorkshire LS22 7TW (GB)
(74) Representative: Glansdorp, Freija Gwendolyn
(86) International application number: PCT/GB2011/052530
(87) International publication number: WO 2012/085559

(56) References cited:
- EP-A2- 0 570 794
- WO-A1-2008/035085
- WO-A2-2011/055139
- GB-A- 2 435 419
- GB-A- 2 456 376
- GB-A- 2 465 633
- JP-A- 5 246 876
- US-A1- 2005 123 620

## Description

### Field of the invention

This invention relates to antibacterial and anti-acne formulations, and to the use of certain combinations of compounds as antibacterial and anti-acne agents.

### Background to the invention

Usnic acid is a naturally occurring dibenzofuran derivative which can be isolated from several species of lichen, in particular *Usnea.* It is also known as usneine, usninic acid and 2,6-diacetyl-7,9-dihydroxy-8,9b-dimethyl-1,3(2H,9bh)-dibenzofurandione.

Usnic acid and usnate salts are known to possess antimicrobial activity, including against Gram-positive bacteria and certain fungi. Usnic acid is also believed to possess antiinflammatory and analgesic activity. The acid and its salts (in particular sodium usnate) have been used in pharmaceuticals, cosmetics and perfumes, both as active ingredients and as preservatives.

Usnates such as copper usnate have been found to be potent against propionibacteria such as *P*. *acnes,* the bacteria implicated in inflammatory acne, and have thus been suggested for use as topical anti-acne agents. However, the sizes and relatively high melting points of usnic acid and usnate salts tend to reduce their skin penetrating ability, and in turn their therapeutic efficacy when applied topically *in vivo*. Often, in order to achieve therapeutically active levels *in vivo*, it is necessary to formulate usnic acid and usnates at relatively high concentrations.

A further difficulty which accompanies the formulation of these actives, however, is their low solubility in most common solvents. There are few materials able to solubilise them at the concentrations required.

It would therefore be desirable to provide antibacterial and anti-acne formulations in which the activity of an usnic acid or usnate active could be enhanced, thus requiring lower overall concentrations of the active agent(s) present.

It has now surprisingly been found that when usnic acid is combined with a copper or bismuth salt, a synergistic effect can be observed on their combined level of antibacterial activity, including against *P. acnes.* As a result, novel antibacterial and anti-acne formulations can be prepared, in particular for topical application, either with improved efficacy and/or containing lower levels of at least one of the active ingredients than would previously have been thought necessary.

### Statements of the invention

According to a first aspect of the present invention there is provided an antibacterial or anti-acne formulation suitable for topical or local application to human skin containing (a) either usnic acid or an usnate salt and (b) a metal salt selected from copper salts, bismuth salts, and mixtures thereof.

Where the component (a) is an usnate salt, the usnate (a) and the metal salt (b) should not be the same compound.

Usnic acid may be used in any one of its tautomeric forms. It may be used in the form of its d-or 1-isomer, or of a racemic mixture. The same applies, *mutatis mutandis*, to usnate salts.

An usnate salt is suitably a pharmaceutically acceptable and/or cosmetically acceptable usnate. It may for example be a metal salt. It may be an alkali metal salt such as sodium usnate. It may be copper usnate (typically copper (II) usnate).

The component (a) may be used in the form of a solvate such as a hydrate. It may be in the form of an oligomer or polymer, for example in which metal ions bind to more than one site in an usnate ion (see for example Takani M et al, 2002, Journal of Inorganic Biochemistry 91: 139-150). It may be either synthetic or naturally occurring (for example it may be used in the form of an extract from a plant source such as *Usneaceae, Evernia, Parmelia* or *Cladonia*)*.*

The usnic acid or usnate may be used in the form of a derivative. In general a "derivative" may be a cosmetically and/or pharmaceutically acceptable derivative (the term "pharmaceutically acceptable" including acceptable for veterinary use). It may be for example a salt, complex or solvate or a so-called "prodrug" form or protected form which reverts to an active form of the relevant compound at an appropriate time on or after administration. In an embodiment of the invention, however, the usnic acid or usnate is present in the form of an underivatised usnic acid molecule or usnate ion as the case may be.

In an embodiment of the invention, the usnic acid or usnate active is selected from usnic acid, sodium usnate, copper usnate and mixtures thereof. In an embodiment, it is selected from usnates (in particular metal usnates) and mixtures thereof, in particular sodium and copper usnates and mixtures thereof. In an embodiment, it is copper usnate. In another embodiment, it is usnic acid.

The component (b) is suitably pharmaceutically and/or cosmetically acceptable. It is a metal salt selected from copper salts, bismuth salts and mixtures thereof. It may be selected from copper salts and mixtures thereof. It may be selected from bismuth salts and mixtures thereof. It may be selected from copper, copper salts and mixtures thereof. It may be selected from bismuth, bismuth salts and mixtures thereof.

In an embodiment of the present invention, the term "metal salt" includes metal complexes.

In an embodiment of the invention, the component (b) is a copper salt or mixture of copper salts. The term "copper salt" includes copper (I), (II) and (III) salts, as well as potentially copper (I), (II) and (III) complexes. In an embodiment the copper salt is a copper (I) (cuprous) or copper (II) (cupric) salt, in particular a copper (II) salt.

In a formulation according to the invention, a copper salt may for instance be selected from copper carboxylates; copper halides; copper sulphadiazine; copper di-usnate; copper sulphate (in particular the pentahydrate); copper nitrate; copper carbonate; copper carbonate hydroxide; copper oxide; copper oxychloride; copper hydroxide; copper acetylacetonate; copper aspartate; copper PCA (pyrrolidone carboxylic acid); copper PCA methylsilanol; copper acetyl tyrosinate methylsilanol; copper acetylmethionate; copper aminoacetylamidoimidazolyl propionate; copper picolinate; copper peptides such as copper tripeptide-1, bis (tripeptide-1) copper acetate, copper ascorbyl phosphate succinoyl tripeptide-34 and alanine/histidine/lysine copper polypeptide HCl; copper ATP; copper DNA; copper amino acid salts (e.g. copper glutamate, copper aspartate and copper glycinate); copper silicates; copper salts of quinolines - especially hydroxyquinolines - and their derivatives (e.g. the copper salt of 8-hydroxyquinoline); copper pyrithione and other copper salts of pyridine thiols; copper phosphates, including for example sodium calcium copper phosphate and copper pyridoxal-5-phosphate; chlorophyllin copper complexes such as sodium copper chlorophyllin; copper chlorophyll; disodium EDTA copper; and mixtures thereof.

Generally speaking a copper salt for use in the formulation of the invention may be either organic or inorganic.

Suitable copper carboxylates include lactate, citrate (including for example disodium cupric citrate, and copper (II) citrate dihydrate), ascorbate, acetate (e.g. either copper (I) acetate or copper (II) acetate), gluconate, laurate, myristate, palmitate, salicylate, aspirinate, stearate, succinate, tartrate, undecylenate, neodecanoate and ricinoleate. A carboxylate may be for example a C2 to C20, a C2 to C18, a C2 to C16, a C2 to C14, a C2 to C12, a C2 to C10, a C2 to C8 or a C2 to C6 carboxylate.

Suitable halides include copper chloride, copper bromide and copper iodide, for example copper (II) chloride or copper (I) iodide.

In an embodiment of the invention, the copper salt may be selected from copper sulphate, copper acetate, copper glycinate, copper gluconate, copper acetylmethionate, copper picolinate, copper citrate, copper PCA (pyrrolidone carboxylic acid), copper PCA methylsilanol, copper acetyl tyrosinate methylsilanol, copper tripeptide-1, copper ATP, copper aspartate, copper DNA, copper carbonate hydroxide, copper pyridoxal-5-phosphate, copper halides such as cupric chloride, disodium cupric citrate, disodium EDTA-copper, bis (tripeptide-1) copper acetate, copper ascorbyl phosphate succinoyl tripeptide-34, chlorophyllin copper complexes, copper aminoacetylamidoimidazolyl propionate, copper chlorophyll; sodium calcium copper phosphate, alanine/histidine/lysine copper polypeptide HCl, and mixtures thereof. In each case the salt may be the copper (II) salt.

In another embodiment, the copper salt may be selected from copper sulphates, carboxylates (in particular C2 to C8 or C2 to C6 carboxylates), halides (especially chlorides) and mixtures thereof. In yet another embodiment, it may be selected from copper (II) sulphate, copper (I) acetate, copper (II) acetate, copper (II) D-gluconate, copper (II) chloride and mixtures thereof; or from copper (II) sulphate, copper (I) acetate, copper (II) D-gluconate, copper (II) chloride and mixtures thereof.

In an embodiment, it may be preferred for the copper salt not to be copper usnate. In other words, when the component (b) is a copper salt, it may be preferred for the formulation to contain, in addition to the usnic acid or usnate (a), a copper salt other than an usnate, even if a copper usnate is also formed in the formulation (between the components (a) and (b)) prior to or on use. The formulation will then contain, in addition to any usnate formed prior to or on use, an additional anion or complexing agent derived from the component (b).

In an embodiment of the invention, the component (b) is a bismuth salt or mixture of bismuth salts. The term "bismuth salt" includes bismuth (III) and (V) salts, as well as potentially bismuth (III) and (V) complexes. In an embodiment, the bismuth salt is a bismuth (III) salt.

A bismuth salt may for instance be selected from bismuth carboxylates, bismuth halides, bismuth sulphadiazine, bismuth sulphate, bismuth nitrate, bismuth subnitrate, bismuth carbonate, bismuth subcarbonate, bismuth oxide, bismuth oxychloride, bismuth hydroxide, bismuth phosphate, bismuth aluminate, bismuth tribromophenate, bismuth thiol, bismuth peptides, bismuth salts of quinolines and their derivatives (e.g. bismuth hydroxyquinolines), bismuth pyrithione and other bismuth salts of pyridine thiols, bismuth amino acid salts such as the glycinate, tripotassium dicitrato bismuthate, and mixtures thereof.

Generally speaking the bismuth salt may be either organic or inorganic. It may be a basic bismuth salt (bismuth subsalt) such as the subsalts referred to above.

Suitable bismuth carboxylates include the salicylate, subsalicylate, lactate, citrate, subcitrate, ascorbate, acetate, dipropylacetate, tartrate, sodium tartrate, gluconate, subgallate, benzoate, laurate, myristate, palmitate, propionate, stearate, undecylenate, aspirinate, neodecanoate and ricinoleate. Of these, basic bismuth salicylate (bismuth subsalicylate) and bismuth citrate may be preferred. A carboxylate may be for example a C2 to C20, a C2 to C18, a C2 to C16, a C2 to C 14, a C2 to C12, a C2 to C10, a C2 to C8 or a C2 to C6 carboxylate.

Suitable halides include bismuth chloride, bismuth bromide and bismuth iodide, in particular bismuth chloride.

In an embodiment, the bismuth salt may be selected from bismuth halides (in particular bismuth chloride), bismuth nitrates, bismuth carboxylates, and mixtures thereof. In another embodiment it may be selected from bismuth subsalicylate, bismuth salicylate, bismuth subgallate, bismuth subcitrate, bismuth citrate, bismuth acetate, bismuth nitrate, bismuth subnitrate and mixtures thereof. In yet another embodiment it may be selected from bismuth subsalicylate, bismuth citrate, bismuth subnitrate and mixtures thereof; or from bismuth subsalicylate, bismuth subnitrate and mixtures thereof; or from bismuth subsalicylate, bismuth citrate and mixtures thereof.

In another embodiment, the bismuth salt may be selected from bismuth halides and mixtures thereof; it may for example be bismuth chloride.

In an embodiment, it may be preferred for the bismuth salt not to be bismuth usnate. In other words, when the component (b) is a bismuth salt, it may be preferred for the formulation to contain, in addition to the usnic acid or usnate (a), a bismuth salt other than an usnate, even if a bismuth usnate is also formed in the formulation (between the components (a) and (b)) prior to or on use. The formulation will then contain, in addition to any usnate formed prior to or on use, an additional anion or complexing agent derived from the component (b).

A metal salt (b) may be used in an at least partially hydrated form, and may thus be formulated in the presence of an aqueous solvent. Alternatively it may be used in the form of a lipid-soluble salt, suitably in the presence of an organic solvent.

In a formulation according to the invention, the metal salt (b) may be used in the form of a suitable derivative. In general a derivative may be a cosmetically and/or pharmaceutically acceptable derivative. It may be for example a complex or solvate, or a so-called "prodrug" form or protected form which reverts to an active form of the relevant compound at an appropriate time on or after administration. In an embodiment of the invention, however, the metal or metal salt (b) is present in the form of the underivatised atomic metal, or of an underivatised ion or complex of the relevant species.

The concentration of the usnic acid or usnate salt (a) in the formulation might suitably be 0.1% w/v or greater, or 0.25% w/v or greater, or 0.5% w/v or greater. Its concentration might be up to 10% w/v, or up to 7.5 or 5% w/v. Its concentration might for example be from 0.5 to 5% w/v, or from 0.5 to 3 or 2% w/v, such as about 1% w/v.

The concentration of the metal salt (b) in the formulation might suitably be 0.01% w/v or greater, or 0.1% w/v or greater. Its concentration might be up to 10% w/v, or up to 5 or 3 or 2% w/v. Its concentration might for example be from 0.1 to 2% w/v, such as about 1% w/v.

The weight ratio of the usnic acid or usnate salt (a) to the metal salt (b) in the formulation may be up to 100:1, for example up to 50:1 or 25:1 or 10:1, or up to 8:1 or 5:1 or 4:1 or 2:1 or 1:1. This ratio may be 1:100 or greater, for example 1:50 or greater or 1:25 or greater or 1:10 or greater, or 1:8 or 1:5 or 1:4 or 1:2 or 1:1 or greater. In cases it may be 2:1 or 4:1 or 5:1 or 8:1 or 10:1 or greater. It may for example be from about 1:8 to 8:1, or from about 1:8 to 4:1, or from about 1:8 to 2:1, or from about 1:4 to 8:1, or from about 1:4 to 4:1, or from about 1:4 to 2:1, or from about 1:2 to 8:1, or from about 1:2 to 4:1, or from about 1:2 to 2:1, or from about 1:8 to 1:1, or from about 1:4 to 1:1, or from about 1:2 to 1:1, or from about 1:8 to 1:2, or from about 1:4 to 1:2, or from about 1:8 to 1:4, or from about 1:1 to 8:1, or from about 1:1 to 4:1, or from about 1:1 to 2:1, or from about 1:2 to 1:8, or from about 1:2 to 1:4, or from about 1:4 to 1:8.

The molecular ratio of the usnic acid or usnate salt (a) to the metal salt (b) in the formulation may be up to 100:1, for example up to 50:1 or 25:1 or 10:1, or up to 8:1 or 5:1 or 4:1 or 2:1 or 1:1. This ratio may be 1:100 or greater, for example 1:50 or greater or 1:25 or greater or 1:10 or greater, or 1:8 or 1:5 or 1:4 or 1:2 or 1:1 or greater. In cases it may be 2:1 or 4:1 or 5:1 or 8:1 or 10:1 or greater. It may for example be from about 1:8 to 8:1, or from about 1:8 to 4:1, or from about 1:8 to 2:1, or from about 1:4 to 8:1, or from about 1:4 to 4:1, or from about 1:4 to 2:1, or from about 1:2 to 8:1, or from about 1:2 to 4:1, or from about 1:2 to 2:1, or from about 1:8 to 1:1, or from about 1:4 to 1:1, or from about 1:2 to 1:1, or from about 1:8 to 1:2, or from about 1:4 to 1:2, or from about 1:8 to 1:4, or from about 1:1 to 8:1, or from about 1:1 to 4:1, or from about 1:1 to 2:1, or from about 1:2 to 1:8, or from about 1:2 to 1:4, or from about 1:4 to 1:8.

In an embodiment, if the metal salt (b) is a copper salt, the molecular ratio of the usnic acid or usnate salt (a) to the copper salt (b) in the formulation is a ratio other than that which would provide the stoichiometric amounts corresponding to the formation of copper usnate. There should therefore be a stoichiometric excess of either the usnate anion or the copper cation present in the formulation, with respect to the *in situ* formation of copper usnate. For example, if the formulation contains (a) usnic acid and (b) a copper(II) salt, the molecular ratio of the usnic acid (a) to the copper (II) salt (b) in the formulation is a ratio other than 2:1.

The formulation according to the invention is suitable for topical or local application to human skin. A formulation which is "suitable for" topical or local application may also be adapted for topical or local application.

The formulation of the invention may be in the form of a fluid, for example a lotion, cream, ointment, varnish, foam, paste, gel or other viscous or semi-viscous fluid, or a less viscous fluid such as might be used in sprays or aerosols. It may take the form of a solution, suspension or emulsion. It may take the form of a solid such as a powder or granules, which may be designed to be added to liquid (e.g. water) prior to use.

In an embodiment the formulation is, or may be, applied to a carrier such as a sponge, swab, brush, pad, tissue, cloth, wipe, skin patch or dressing (which includes a bandage, plaster, skin adhesive or other material designed for application to a tissue surface), to facilitate its administration.

For use in the treatment of a skin or skin structure condition, in particular acne, the formulation may for example take the form of a lotion, cream, ointment, varnish, foam, paste or gel; alternatively it may be, or be capable of being, applied to a carrier of the type described above.

A formulation according to the invention may be intended for pharmaceutical (which includes veterinary but is preferably human) use, and/or for cosmetic or other non-medical care purposes (for example, to cleanse the skin, or to improve the appearance, feel or smell of the skin).

A formulation according to the invention may contain excipients and other additives known for use in pharmaceutical or veterinary formulations. Suitable excipients for use in formulations designed for topical or local application will be well known to those skilled in the art. Those included will depend on the intended mode and site of application for the formulation. In the context of formulations for topical application to the skin, examples may for instance be found in Williams' Transdermal and Topical Drug Delivery (Pharmaceutical Press, 2003) and other similar reference books. See also Date, AA et al, Skin Pharmacol Physiol, 2006, 19(1): 2-16 for a review of topical drug delivery strategies, and also Skin Delivery Systems, 2006, John J Wille, Ed, Blackwell Publishing; Textbook of Cosmetic Dermatology, 2004, 3rd edition, Robert Baran, Howard I Maibach, Taylor & Francis; and Skin Care Beyond the Basics, 2001, Mark Lees, Milady.

The usnic acid or usnate (a) and the metal salt (b) may each independently be present in the form of a solution or suspension, the term "suspension" including emulsions, micellar systems and other multi-phase dispersions.

The excipient(s) used may be suitable for targeting or controlling release of the formulation, or of a component of the formulation, at the intended site of administration, for instance to target a desired site and/or time of delivery. Such excipients may for instance target the formulation to a region of the skin, for example the stratum corneum or the pilosebaceous follicles, or to hair follicles. They may delay or otherwise control release of the formulation over a particular time period. Either or both of the components (a) and (b) may be microencapsulated: suitable encapsulating entities include liposomes, niosomes, aspasomes, cubosomes, microsponges, microemulsions, hydrogels and solid lipid nanoparticles. Particularly suitable liposomes, for topical application to the skin, are those made from stratum corneum lipids, e.g. ceramides, fatty acids or cholesterol.

Where the formulation is intended for topical application to the skin, examples of suitable additives include emollients, moisturisers, perfumes, antioxidants, preservatives, stabilisers, gelling agents and surfactants; others may be found in Williams' *Transdermal and Topical Drug Delivery* (see above). For the treatment of acne, however, it may be preferred for the formulation not to contain an emollient.

Such a formulation may further contain additional active agents such as antimicrobial (in particular antibacterial) agents. For example, it may contain one or more agents selected from anti-acne agents, keratolytics, comedolytics, agents capable of normalising keratinocyte and/or sebocyte function, anti-inflammatories, anti-proliferatives, antibiotics, anti-androgens, sebostatic/sebosuppressive agents, anti-pruritics, immunomodulators, agents which promote wound healing, additional antimicrobial agents, sunscreens, skin lightening agents, anti-ageing substances, and mixtures thereof.

In this context an additional antimicrobial agent may be selected from biocides, disinfectants, antiseptics, antibiotics, bacteriophages, enzymes, anti-adhesins, immunoglobulins, antimicrobially active antioxidants, and mixtures thereof; it may be active as a bactericide, in particular against propionibacteria. It may however be preferred for the components (a) and (b) to be the only active agents in the formulation, or at least to be the only antimicrobially or antibacterially active agents and/or the only anti-acne active agents.

A formulation according to the invention may be incorporated into, and hence applied in the form of, another product such as a cosmetic; a skin care preparation (for example a skin cleanser, toner or moisturiser); a deodorant or anti-perspirant; a cleansing preparation (for example a facial wash); a pharmaceutical (which includes veterinary) preparation; a cosmeceutical preparation; a toiletry product (for instance a bath or shower additive or a soap); or a laundry or other fabric treatment product. The formulation may be, or be incorporated into, a wash-off skin treatment product such as a skin cleanser, or in particular a leave-on skin treatment product.

The invention thus provides, according to a second aspect, a product which incorporates an antibacterial or anti-acne formulation according to the first aspect.

In an embodiment of this second aspect, the product may carry the antibacterial or anti-acne formulation. The product may for instance be coated with the formulation, or it may be impregnated with the formulation, or the formulation may be contained in any location in or on the product.

A formulation according to the invention may be prepared *in situ,* at or immediately before its point of use, for instance its application to the skin or another surface. Thus according to a third aspect, the present invention provides a kit for preparing an antibacterial or anti-acne formulation, the kit comprising sources of (a) usnic acid or an usnate and (b) a metal salt selected from copper salts, bismuth salts and mixtures thereof, together with instructions for combining the two components so as to make the formulation at or before the point of intended use, and/or for the co-administration of the two components to a surface such as the skin, wherein if the component (a) is an usnate, the components (a) and (b) are not the same compound. The two components may each be present in a suitable respective vehicle.

According to one embodiment, the formulation or kit of the invention may contain the two components (a) and (b), each encapsulated (for instance microencapsulated) in a separate delivery vehicle; this might for instance allow their release, and hence their contact with one another, only at the intended site of administration.

A formulation according to the invention may be marketed with an indication that it has antibacterial and/or anti-acne activity, or enhanced antibacterial and/or anti-acne activity. The marketing of such a formulation may include an activity selected from (i) enclosing the formulation in a container or package that comprises the relevant indication; (ii) packaging the formulation with a package insert that comprises the indication; (iii) providing the indication in a publication that describes the formulation; and (iv) providing the indication in a commercial which is aired for instance on the radio, television or internet. The activity or enhancement may be attributed, in such an indication, at least partly to the presence of either or both of the components (a) and (b). The invention may involve assessing the activity of the formulation during or after its preparation, for instance against one or more of the pathogens referred to below. It may involve assessing the activity both before and after incorporation of the usnic acid or usnate (a) and/or the metal salt (b), for example so as to confirm that either or both contribute to the antibacterial or anti-acne activity of the formulation.

A fourth aspect of the invention provides a method for preparing an antibacterial or anti-acne formulation, which method involves mixing together (a) usnic acid or an usnate and (b) a metal salt selected from copper salts, bismuth salts and mixtures thereof, wherein if the component (a) is an usnate, the components (a) and (b) are not the same compound, suitably together with a cosmetically and/or pharmaceutically acceptable vehicle.

In the formulation of the invention, the combination of the components (a) and (b) is typically present as an active (i.e. antibacterially active) agent. The combination may be present as an anti-acne agent (i.e. as an agent which is active against acne (which includes against a symptom and/or a cause of acne and/or against one or more micro-organisms associated with acne)).

It is possible that the antibacterial and/or anti-acne activity of a combination of components (a) and (b) may be at least partly due to the formation of a reaction product which itself has antibacterial and/or anti-acne activity. The invention may thus embrace an antibacterial or anti-acne formulation containing a reaction product formed between (a) usnic acid or an usnate and (b) a metal salt selected from copper salts, bismuth salts and mixtures thereof; this reaction product may be formed *in situ* immediately prior to, or at the point of, use.

In the present context, antibacterial activity embraces activity against both Gram-positive and Gram-negative bacteria, in particular Gram-positive bacteria. It may be growth inhibitory activity or more preferably biocidal (i.e. lethal to the relevant organism). It may comprise activity against sessile and/or planktonic bacteria; in other words, it may comprise activity against bacteria which are growing as or in a biofilm. In the context of this invention, activity against a particular species of micro-organism may be taken to mean activity against at least one, or against two or more, strains of that species.

Antibacterial activity may be or include the ability to disrupt and/or suppress biofilm formation by the relevant organism. In the present context, the disruption of biofilm formation embraces any negative effect on the ability of a micro-organism to form, maintain or exist in a biofilm, and/or on a biofilm already formed by the organism. Thus, it may involve reducing the amount of a previously formed biofilm, and/or impairing such a biofilm. It may involve killing or inhibiting sessile micro-organisms within a biofilm.

Suppression of biofilm formation embraces any degree of impairment (including complete prevention) of the ability of a micro-organism to form, or more typically to co-aggregate with, a biofilm. It thus embraces total or partial impairment, including reducing the amount and/or strength of biofilm which the organism is able to form and/or the speed with which it is able to do so. It may involve preventing or reducing the growth or the rate of growth of an existing biofilm formed by the organism.

In an embodiment, the formulation of the invention is active against one or more bacteria associated with acne, in particular propionibacteria such as *P. acnes* and in some instances *P*. *granulosum*.

The formulation may be active against bacteria, in particular propionibacteria, which are wholly or partially resistant to one or more antibiotics, for instance those which are in common clinical use. For example it may be active against one or more macrolide-lincosamide-streptogramin (MLS) resistant and/or macrolide-lincosamide-streptogramin-ketolide (MLSK) resistant strains of bacteria. In particular it may be active against one or more erythromycin-resistant, clindamycin-resistant and/or tetracycline-resistant strains of bacteria, for example *P*. *acnes* strains, the term tetracycline here referring to the class of antibiotics including for example minocycline and doxycycline as well as the specific antibiotic known as tetracycline.

According to a fifth aspect of the present invention, there is provided a formulation containing (a) usnic acid or an usnate and (b) a metal salt selected from copper salts, bismuth salts and mixtures thereof, wherein if the component (a) is an usnate, the components (a) and (b) are not the same compound, for use in the treatment of a condition which is caused by, transmitted by and/or exacerbated by (in particular either caused or transmitted by, more particularly caused by) bacterial activity. The bacterial activity may be propionibacterial activity. It may be activity by one or more coryneform bacteria.

In the context of the present invention, treatment of a condition encompasses both therapeutic and prophylactic treatment, in either a human or animal but in particular a human. It may involve complete or partial eradication of the condition, removal or amelioration of associated symptoms, arresting subsequent development of the condition, and/or prevention of, or reduction of risk of, subsequent occurrence of the condition. It will typically involve use of the components (a) and (b) as an antibacterial and/or anti-acne combination.

Treatment may involve use of the formulation against bacterial biofilm formation. The biofilm may be formed by, and/or may be associated with, a propionibacterium, in particular a cutaneous propionibacterium.

In general the treatment may be administered by any appropriate route, for instance transdermally or topically. In an embodiment, it is administered locally. In an embodiment, it is administered topically.

In an embodiment of the fifth aspect of the invention, the formulation is for use against one or more propionibacteria, in particular against one or more cutaneous propionibacteria, more particularly against one or more bacteria associated with acne, such as *P. acnes* and in some instances *P. granulosum*. Thus, the formulation may be for use in the treatment of acne.

Acne is a multifactorial disease of the pilosebaceous follicles of the face and upper trunk, characterised by a variety of inflamed and non-inflamed lesions such as papules, pustules, nodules and open and closed comedones. Its treatment can therefore encompass the treatment (which embraces prevention or reduction) of any of these symptoms, and references to use as an anti-acne agent may be construed accordingly. In particular, the treatment of acne encompasses the treatment (including prevention) of lesions and/or scarring associated with acne. It also encompasses the inhibition of propionibacterial activity which could cause or be otherwise associated with acne or its symptoms. In the context of the present invention, it may in particular be the treatment of inflamed acne lesions.

In general, the present invention will be used for the treatment of symptoms which are directly due to acne rather than for instance infections which may arise as a consequence of treating acne with other actives such as antibiotics, and/or secondary infections caused by opportunistic pathogens, which can arise in skin already affected by acne. It will not generally be used for the treatment of symptoms which are acne-like but which are not etiologically the same as acne, for instance skin rashes caused by treatment with other medicaments such as epidermal growth factor receptor (EGFR) inhibitors.

In another embodiment, the formulation of the invention may be for use against an opportunistic infection which is caused, transmitted and/or exacerbated by (in particular caused by) propionibacteria, for instance an infection associated with an indwelling surgical device (a prosthetic joint, shunt, stent or catheter, for example). In the latter case, the device may be coated or impregnated with the formulation. The formulation may be for use in treating an infected wound, burn or ulcer. It may be for use against any other infection or condition which involves or can involve propionibacteria, for example an eye infection such as endophthalmitis, or in cases body odour.

In a further embodiment, the formulation may be for use in the treatment of skin and skin structure infections such as superficial wounds, atopic dermatitis or infected eczema.

In an embodiment of the fifth aspect of the invention, the formulation may be prepared *in situ,* at or immediately before the point of administration. This aspect of the invention thus pertains to any use of (a) usnic acid or an usnate and (b) a metal salt selected from copper salts, bismuth salts and mixtures thereof, in the treatment of a bacterial condition such as acne, the two components being administered either simultaneously or sequentially.

A sixth aspect of the invention provides the use of a formulation containing (a) usnic acid or an usnate and (b) a metal salt selected from copper salts, bismuth salts and mixtures thereof, wherein if the component (a) is an usnate, the components (a) and (b) are not the same compound, in the manufacture of a medicament (typically a formulation) for the treatment of a condition affecting the human or animal body, which condition is caused by, transmitted by and/or exacerbated by (in particular either caused or transmitted by, more particularly caused by) bacterial activity. The components (a) and (b) will typically be used as an antibacterial combination in the manufacture of the medicament, and/or as an anti-acne combination.

The invention further provides, according to a seventh aspect, the use together of (a) usnic acid or an usnate and (b) a metal salt selected from copper salts, bismuth salts and mixtures thereof, wherein if the component (a) is an usnate, the components (a) and (b) are not the same compound, as a combined antibacterial agent, and/or as a combined anti-acne agent, for example in the manufacture of an antibacterial or anti-acne formulation.

An eighth aspect provides the use of a formulation according to the first aspect of the invention, for non-therapeutic purposes. In an embodiment of this eighth aspect, the formulation is used as an anti-acne or in particular a skin care agent for non-therapeutic purposes, for example for cosmetic purposes such as to improve the appearance, feel or smell of the skin.

A ninth aspect provides a method for controlling the growth of a bacterium, the method comprising applying, to a non-living area or surface which is infected or suspected to be infected or capable of becoming infected with the bacterium, a formulation according to the first aspect of the invention. Again the components (a) and (b) may be applied simultaneously or sequentially. They are suitably applied topically or locally. They may in particular be applied to an area or surface which is infected with the bacterium.

The bacterium may be one of those identified above in connection with the fifth aspect of the invention, in particular a propionibacterium.

In accordance with then ninth aspect of the invention, "applying" a formulation to a surface embraces immersing the surface in the formulation.

The formulation may be applied to a non-living area or surface such as in a hospital or surgery. Thus the invention may be used to disinfect work surfaces, surgical or other instruments (including implants or prostheses) or other devices against bacteria. It may be used to treat protective clothing such as surgical gloves, clothing or bedding. In an embodiment, it may be used to treat an implant or other device which is intended for use within the body. "Controlling the growth" of a bacterium embraces inhibiting or preventing its growth, whether completely or partially, as well as killing either completely or partially a culture of the bacterium. It also embraces reducing the risk of subsequent growth of the bacterium in or on the area or surface being treated. It may embrace reducing the risk of transmission of the bacterium from the area or surface being treated to another area or surface and/or living body. The method of the invention may thus be used to treat an existing occurrence of the bacterium or to prevent a potential subsequent occurrence. Controlling the growth of a bacterium may also embrace the disruption and/or suppression of biofilm formation by the organism.

A tenth aspect of the invention provides the use of usnic acid or an usnate, in an antibacterial or anti-acne formulation containing a metal salt selected from copper salts, bismuth salts and mixtures thereof, wherein if the component (a) is an usnate, the components (a) and (b) are not the same compound, for the purpose of increasing the antibacterial and/or anti-acne activity of the formulation and/or of reducing the amount of the metal salt in the formulation without or without undue loss of antibacterial or anti-acne activity.

An increase in antibacterial or anti-acne activity may be as compared to that of the metal salt alone, at the same concentration as used when combined with the usnic acid or usnate. Ideally the increase is as compared to the sum of the activities of the usnic acid or usnate and the metal salt individually, again at the same respective concentrations as used when the two are combined.

A reduction in the amount of the metal salt in the formulation may be as compared to the amount which would otherwise have been used in the formulation in order to achieve a desired level of activity, in particular in order to have acceptable efficacy in the context of its intended use. The reduction may be manifested by reduced side effects which would otherwise have been observed during use of the formulation, for example local irritation and/or undesirable systemic absorption of the metal salt; it may be manifested by a change in the properties of the formulation, for example by a reduction in colour. According to the invention, the usnic acid or usnate may therefore be used for the dual purposes of reducing an undesired property of a formulation containing a metal salt selected from copper salts, bismuth salts and mixtures thereof, without or without undue loss of antibacterial or anti-acne activity.

The usnic acid or usnate may be used without any reduction in antibacterial or anti-acne activity compared to the level exhibited by the formulation prior to addition of the usnic acid or usnate. It may be used to give an increase in antibacterial or anti-acne activity, in particular *in vivo*. It may however be used to reduce the amount of the metal salt present, and/or its associated side effects, and/or to alter a property of the formulation, whilst maintaining the antibacterial or anti-acne activity of the resultant formulation at a level, albeit lower than that which it would otherwise have exhibited, which is still acceptable in the context of its intended use.

An eleventh aspect of the invention provides the use of a metal salt selected from copper salts, bismuth salts and mixtures thereof, in an antibacterial or anti-acne formulation containing usnic acid or an usnate according to the first aspect of the invention, for the purpose of increasing the antibacterial and/or anti-acne activity of the formulation and/or of reducing the amount of the usnic acid or usnate in the formulation without or without undue loss of antibacterial or anti-acne activity. The above comments regarding then tenth aspect of the invention apply *mutatis mutandis* to the eleventh aspect.

Throughout the description and claims of this specification, the words "comprise" and "contain" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise. Preferred features of each aspect of the invention may be as described in connection with any of the other aspects. Other features of the invention will become apparent from the following examples. Generally speaking the invention extends to any novel one, or any novel combination, of the features disclosed in this specification (including any accompanying claims and drawings). Thus features, integers, characteristics, compounds, chemical moieties or groups described in conjunction with a particular aspect, embodiment or example of the invention are to be understood to be applicable to any other aspect, embodiment or example described herein unless incompatible therewith. Moreover unless stated otherwise, any feature disclosed herein may be replaced by an alternative feature serving the same or a similar purpose.

Where upper and lower limits are quoted for a property, for example for the concentration of an active agent in a formulation, then a range of values defined by a combination of any of the upper limits with any of the lower limits may also be implied.

The present invention will now be further described with reference to the following nonlimiting examples.

### Detailed description

Experimental tests were conducted to determine the antibacterial activity of formulations containing usnic acid and either a copper or a bismuth salt.

The test organism used was *Propionibacterium acnes* NCTC 737. This is a propionibacterial strain and is the type strain of the genus; it is fully susceptible to antibiotics. The propionibacteria are clinically significant due to their involvement in acne. They are also opportunistic pathogens in compromised hosts. Activity observed against these micro-organisms is expected to be a good predictor of activity against acne.

The propionibacteria were cultured and maintained on Wilkins-Chalgren Anaerobe Medium (agar and broth) at pH 6.0; all cultures were incubated anaerobically at 37°C for 72 hours.

The following "fixed ratio combination MIC/MBC assay" was carried out to assess antimicrobial activity against the test organism.

The method used a sterile 96-well microtitre plate, capable of holding about 200 µl of liquid per well. The wells contained liquid culture medium and ranges of decreasing concentrations of the relevant test compounds alone in doubling dilutions (e.g. 1000, 500, 250, 125...µg/ml, etc.. down to 0.49 µg/ml) or doubling dilutions of a combination of two compounds mixed initially at a fixed concentration ratio (based on molarity in this instance). The culture medium was as described above.

The wells were inoculated with a liquid suspension of freshly grown micro-organism and incubated under the conditions described above. After incubation, the microtitre plate was examined visually (with the aid of a light box) for cloudiness in each well, which would indicate microbial growth. The minimum inhibitory concentration (MIC) value was recorded as the lowest concentration of test compound alone, or the lowest concentration of the mixture of the two test compounds, required to inhibit microbial growth, i.e. where the liquid in the well remained clear.

The assays were conducted in triplicate and included both negative (culture medium with no micro-organisms) and positive (culture medium plus diluting solvent(s) plus micro-organism) controls.

Following the MIC assay, a 5 µl sample was withdrawn from the first microtitre well that showed positive growth and from each of the subsequent wells that showed no growth. These samples were then individually sub-cultured on antibiotic-free agar medium, under the incubation conditions described above. Following incubation they were examined visually for microbial growth. The minimum bactericidal concentration (MBC) was taken to be the lowest test compound concentration alone, or the lowest concentration of the mixture of the two test compounds, for which the incubated sample showed no growth.

A fractional inhibitory concentration index (FICI) or fractional bactericidal concentration index (FBCI) was then calculated for each compound in the relevant mixture, and these two indices were added together to give an overall FICI or FBCI to indicate the mode of interaction.

Thus for each mixture (of two compounds A and B) which was tested, the FIC for compound A (FIC_{A}) = MIC for (A + B) / MIC for A alone. Similarly the FIC for compound B (FIC_{B}) = MIC for (A + B) / MIC for B alone. The overall FICI = FIC_{A} + FIC_{B}. FBCs and FBCIs were calculated in the same manner, using recorded MBC values.

An FICI or FBCI of 0.5 or less was taken to indicate synergy; a value greater than 0.5 up to 4.0 an indifferent effect; and values greater than 4.0 antagonism (i.e. the two compounds counter one another's activity, leading overall to a diminished antimicrobial effect) (see Odds FC, "Synergy, antagonism, and what the chequerboard puts between them", J Antimicrob Chemother, 2003; 52:1).

In instances where the highest assayed concentration of a test compound failed to inhibit and/or to kill the micro-organism, MIC and/or MBC values for that compound were assumed to be double the highest assayed concentration: this enabled calculation of finite FICI and/or FBCI values.

### Example 1 - activity against P. acnes (copper (II) sulphate)

This example used *Propionibacterium acnes* NCTC 737 as the test organism. Fixed ratio combination MIC/MBC assays, as described above, were carried out using the test compounds usnic acid and copper (II) sulphate (both sourced from Sigma-Aldrich, UK). The usnic acid was dissolved in DMSO and the copper sulphate in deionised water. All experiments were performed in triplicate.

The results are shown in Table 1 below. Where two figures are shown the first corresponds to the end point of usnic acid (UA) and the second to that of the metal salt in the mixture.

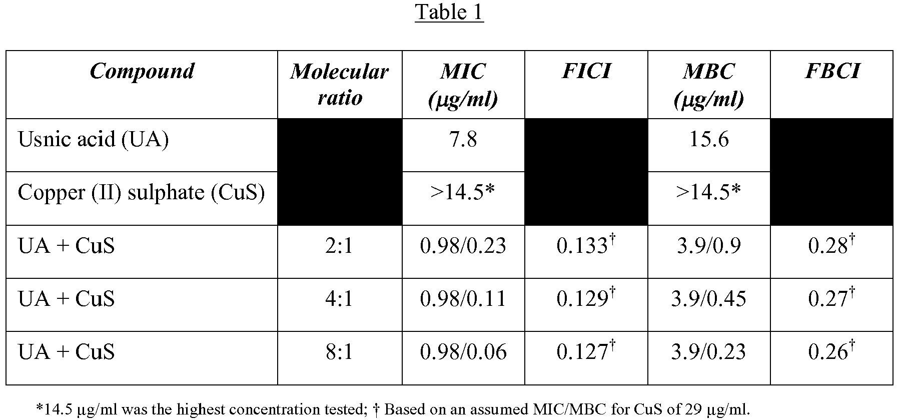

These data show that the usnic acid alone, and to a lesser extent the copper salt, is active against *P. acnes* NCTC 737. Surprisingly, when the usnic acid and the copper salt are combined (up to a molecular ratio of 8:1 UA to CuS), the data demonstrate a synergistic (FICI and FBCI recorded at ≤0.5) antimicrobial interaction between the two. The presence of even low levels of the copper salt significantly decreases both the MIC and the MBC of usnic acid when compared to those recorded for the acid alone. This indicates the likely activity of the invented formulations as anti-acne agents, the propionibacteria being implicated in acne.

### Example 2 - activity against P. acnes (other copper salts)

This example also used *Propionibacterium acnes* NCTC 737 as the test organism. Fixed ratio combination MIC/MBC assays, as described above, were carried out using the test compounds usnic acid, copper (II) acetate, copper (I) acetate, copper (II) D-gluconate and copper (II) chloride (all sourced from Sigma-Aldrich, UK). The usnic acid was dissolved in DMSO and the copper salts in deionised water. All experiments were performed in triplicate.

The results are shown in Tables 2 to 5 below. Data were collated from a number of experiments. Again, where two figures are shown in the tables, the first corresponds to the end point of usnic acid (UA) and the second to that of the metal salt in the mixture.

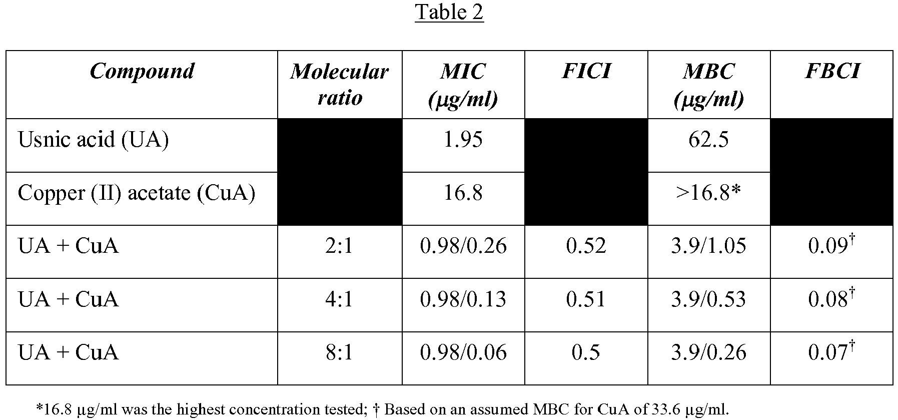

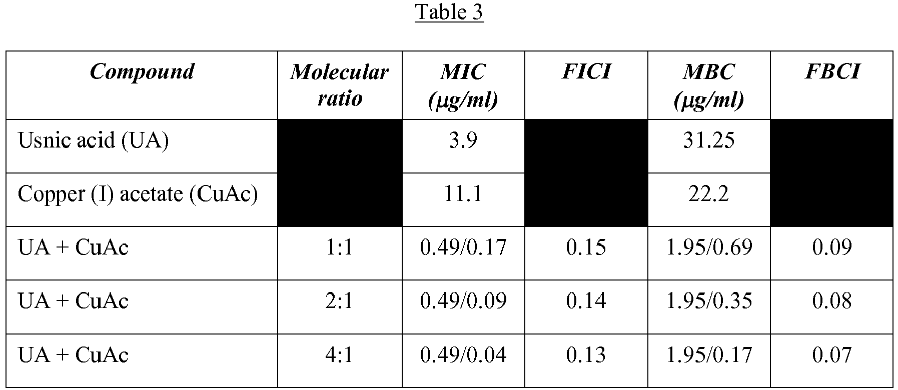

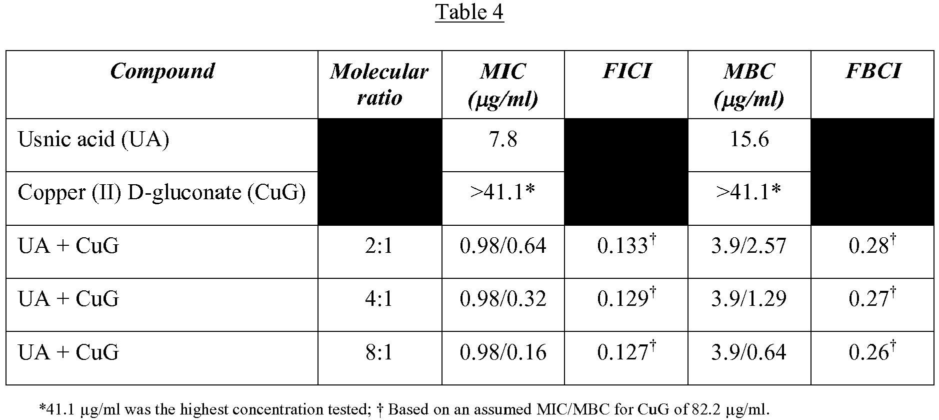

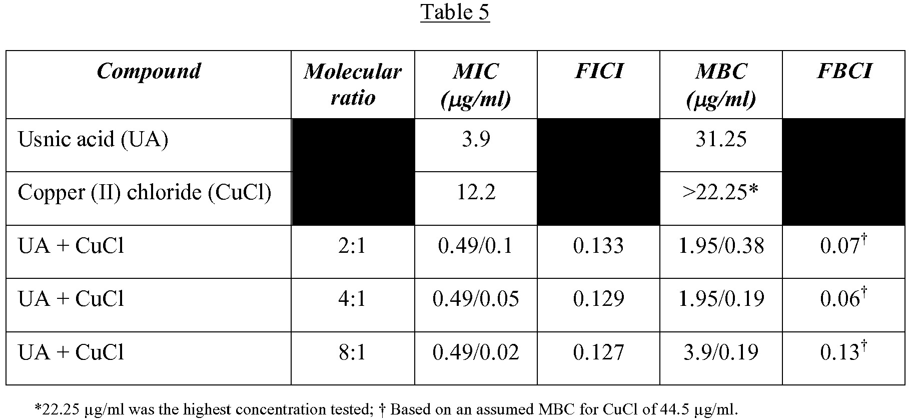

Surprisingly, when the usnic acid and the copper salts are combined at various molecular ratios, the data demonstrate a synergistic (FICI and FBCI recorded at ≤0.5) antimicrobial interaction between the two, with the exception of the copper (II) acetate FICI. The presence of even low levels of the copper salts significantly decreases both the MIC and the MBC of usnic acid when compared to those recorded for the acid alone. This further indicates the likely activity of the invented formulations as anti-acne agents.

### Example 3 - activity against P. acnes (bismuth (III) chloride)

Fixed ratio combination MIC/MBC assays, as described above, were carried out using the test compounds usnic acid and bismuth (III) chloride (both sourced from Sigma-Aldrich, UK). Both were dissolved in DMSO. All experiments were performed in triplicate.

The results are shown in Table 6 below. Where two figures are shown the first corresponds to the end point of usnic acid (UA) and the second to that of the metal salt in the mixture

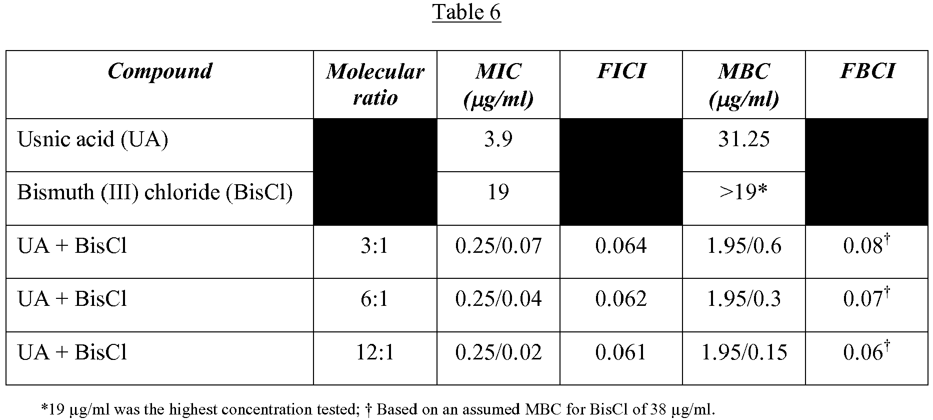

These data show that both the usnic acid and the bismuth salt are active against *P. acnes* NCTC 737. When the two compounds are combined, up to a molecular ratio of 12:1 UA to BisCl, the data demonstrate a synergistic (FICI and FBCI recorded at ≤0.5) antimicrobial interaction between the two. The presence of even low levels of the bismuth salt significantly decreases both the MIC and the MBC of usnic acid when compared to those recorded for the acid alone. This indicates the likely activity of a formulation containing both usnic acid and a bismuth salt, as an anti-acne agent.

### Example 4 - topical anti-acne formulations

The results from Examples 1 to 3 show that the combination of usnic acid with either a copper or a bismuth salt can be an effective antibacterial agent against the bacteria associated with acne. This can be of use in preparing antibacterial formulations, in particular for topical application to the skin, for either prophylactic or therapeutic use in any context where such bacteria are thought to be involved as possible sources of infection. More specifically, it can be of use in preparing anti-acne formulations, again suitably for topical use.

A topical formulation for use in treating acne may for example be prepared by combining usnic acid with a copper salt such as copper sulphate, or with a bismuth salt such as bismuth chloride, in a suitable fluid vehicle and optionally together with conventional additives. Such vehicles and additives may be for instance as found in Williams' Transdermal and Topical Drug Delivery, Pharmaceutical Press, 2003 and other similar reference books, and/or in Rolland A et al, "Site-specific drug delivery to pilosebaceous structures using polymeric microspheres", Pharm Res 1993; 10: 1738-44; Mordon S et al, "Site-specific methylene blue delivery to pilosebaceous structures using highly porous nylon microspheres: an experimental evaluation", Lasers Surg Med 2003; 33: 119-25; and Alvarez-Roman R et al, "Skin penetration and distribution of polymeric nanoparticles", J Controlled Release 2004; 99: 53-62.

The formulation may be prepared and administered using known techniques. It may for example take the form of a cream, lotion or in particular a gel.

The concentrations of the two active agents may be in the ranges described above, and will be determined based on the intended use of the formulation, its intended mode of administration and the activities of the particular chosen active agents. Suitably, the formulation is administered topically to acne-affected skin.

Even in cases where a combination of usnic acid or an usnate with a copper or bismuth salt has an indifferent, as opposed to synergistic, antibacterial activity compared to that of the individual compounds, this can be of considerable benefit when preparing formulations for topical use. One of the compounds may be used to replace a proportion of the other, thus lowering any side effects and/or other undesirable properties of the combination without undue loss of antibacterial activity.

## Claims

1. An antibacterial or anti-acne formulation suitable for topical or local application to human skin containing (a) either usnic acid or an usnate and (b) a metal salt selected from copper salts, bismuth salts, and mixtures thereof, wherein if the component (a) is an usnate, the components (a) and (b) are not the same compound.

2. A formulation according to claim 1, wherein the component (a) is usnic acid.

3. A formulation according to claim 1 or claim 2, wherein the component (b) is selected from copper salts and mixtures thereof.

4. A formulation according to claim 1 or claim 2, wherein the component (b) is selected from bismuth salts and mixtures thereof.

5. A formulation according to any one of the preceding claims, which is in the form of a fluid.

6. A formulation according to claim 5, which is in the form of a lotion, cream, ointment, varnish, foam, paste or gel.

7. A product which incorporates an antibacterial or anti-acne formulation according to any one of the preceding claims.

8. A kit for preparing an antibacterial or anti-acne formulation, the kit comprising sources of (a) usnic acid or an usnate and (b) a metal salt selected from copper salts, bismuth salts, and mixtures thereof, together with instructions for combining the two components so as to make the formulation at or before the point of intended use, and/or for the co-administration of the two components to a surface such as the skin, wherein if the component (a) is an usnate, the components (a) and (b) are not the same compound.

9. A formulation containing (a) usnic acid or an usnate and (b) a metal salt selected from copper salts, bismuth salts, and mixtures thereof, wherein if the component (a) is an usnate, the components (a) and (b) are not the same compound, for use in the treatment of a condition which is caused by, transmitted by and/or exacerbated by bacterial activity.

10. The formulation for use according to claim 9, wherein the condition is acne.

11. The formulation for use according to claim 9 or claim 10, wherein the treatment is administered topically.

12. A method for controlling the growth of a bacterium, the method comprising applying, to a non-living area or surface which is infected or suspected to be infected or capable of becoming infected with the bacterium, a formulation containing (a) usnic acid or an usnate and (b) a metal salt selected from copper salts, bismuth salts, and mixtures thereof, wherein if the component (a) is an usnate, the components (a) and (b) are not the same compound.

## Patentansprüche

1. Antibakterielle oder Anti-Akne-Formulierung, welche sich zur topischen oder lokalen Anwendung auf der menschlichen Haut eignet, enthaltend (a) entweder Usninsäure oder ein Usnat und (b) ein Metallsalz, welches ausgewählt ist aus Kupfersalzen, Bismutsalzen und Mischungen davon, wobei, wenn der Bestandteil (a) ein Usnat ist, die Bestandteile (a) und (b) nicht dieselbe Verbindung sind.

2. Formulierung nach Anspruch 1, wobei der Bestandteil (a) Usninsäure ist.

3. Formulierung nach Anspruch 1 oder Anspruch 2, wobei der Bestandteil (b) ausgewählt ist aus Kupfersalzen und Mischungen davon.

4. Formulierung nach Anspruch 1 oder Anspruch 2, wobei der Bestandteil (b) ausgewählt ist aus Bismutsalzen und Mischungen davon.

5. Formulierung nach einem der vorhergehenden Ansprüche, welche in Form eines Fluids vorliegt.

6. Formulierung nach Anspruch 5, welche als Lotion, Creme, Salbe, Lack, Schaum, Paste oder Gel vorliegt.

7. Eine antibakterielle oder eine Anti-Akne-Formulierung nach einem der vorhergehenden Ansprüche enthaltendes Produkt.

8. Kit zur Herstellung einer antibakteriellen oder Anti-Akne-Formulierung, umfassend Quellen für (a) Usninsäure oder ein Usnat und (b) ein Metallsalz, welches ausgewählt ist aus Kupfersalzen, Bismutsalzen und Mischungen davon, zusammen mit Anweisungen zum Kombinieren der beiden Bestandteile zur Herstellung der Formulierung zum oder vor dem Zeitpunkt der beabsichtigten Anwendung und/oder zur gemeinsamen Verabreichung der beiden Bestandteile auf eine Oberfläche wie die Haut, wobei, wenn der Bestandteil (a) ein Usnat ist, die Bestandteile (a) und (b) nicht dieselbe Verbindung sind.

9. Formulierung, enthaltend (a) Usninsäure oder ein Usnat und (b) ein Metallsalz, welches ausgewählt ist aus Kupfersalzen, Bismutsalzen und Mischungen davon, wobei, wenn der Bestandteil (a) ein Usnat ist, die Bestandteile (a) und (b) nicht dieselbe Verbindung sind, zur Verwendung bei der Behandlung eines durch bakterielle Aktivität verursachten, übertragenen und/oder verstärkten Zustands.

10. Formulierung zur Verwendung nach Anspruch 9, wobei der Zustand Akne ist.

11. Formulierung zur Verwendung nach Anspruch 9 oder Anspruch 10, wobei die Behandlung als topische Verabreichung erfolgt.

12. Verfahren zur Steuerung des Wachstums eines Bakteriums, umfassend das Auftragen einer Formulierung, welche (a) Usninsäure oder ein Usnat und (b) ein Metallsalz enthält, welches ausgewählt ist aus Kupfersalzen, Bismutsalzen und Mischungen davon, wobei, wenn der Bestandteil (a) ein Usnat ist, die Bestandteile (a) und (b) nicht dieselbe Verbindung sind, auf einen unbelebten Bereich oder eine unbelebte Oberfläche, welche mit dem Bakterium infiziert ist, in Verdacht steht infiziert zu sein oder in der Lage ist, infiziert zu werden.

## Revendications

1. Formulation antibactérienne ou anti-acnéique appropriée pour une application topique ou locale sur la peau humaine, contenant (a) de l'acide usnique ou un usnate et (b) un sel métallique sélectionné parmi les sels de cuivre, les sels de bismuth et leurs mélanges, dans laquelle si le composant (a) est un usnate, les composants (a) et (b) ne sont pas le même composé.

2. Formulation selon la revendication 1, dans laquelle le composant (a) est l'acide usnique.

3. Formulation selon la revendication 1 ou 2, dans laquelle le composant (b) est sélectionné parmi les sels de cuivre et leurs mélanges.

4. Formulation selon la revendication 1 ou 2, dans laquelle le composant (b) est sélectionné parmi les sels de bismuth et leurs mélanges.

5. Formulation selon l'une quelconque des revendications précédentes, qui est sous la forme d'un fluide.

6. Formulation selon la revendication 5, qui est sous la forme d'une lotion, d'une crème, d'une pommade, d'un vernis, d'une mousse, d'une pâte ou d'un gel.

7. Produit qui incorpore une formulation antibactérienne ou anti-acnéique selon l'une quelconque des revendications précédentes.

8. Kit pour la préparation d'une formulation antibactérienne ou anti-acnéique, le kit comprenant des sources (a) d'acide usnique ou d'usnate et (b) de sel métallique sélectionné parmi les sels de cuivre, les sels de bismuth et leurs mélanges, conjointement avec des instructions pour combiner les deux composants afin de préparer la formulation à ou avant l'endroit d'utilisation prévue, et/ou pour la co-administration des deux composants à une surface telle que la peau, dans lequel si le composant (a) est un usnate, les composants (a) et (b) ne sont pas le même composé.

9. Formulation contenant (a) de l'acide usnique ou un usnate et (b) un sel métallique sélectionné parmi les sels de cuivre, les sels de bismuth et leurs mélanges, dans laquelle si le composant (a) est un usnate, les composants (a) et (b) ne sont pas le même composé, pour son utilisation dans le traitement d'une affection qui est provoquée, transmise et/ou exacerbée par une activité bactérienne.

10. Formulation pour son utilisation selon la revendication 9, dans laquelle l'affection est l'acné.

11. Formulation pour son utilisation selon la revendication 9 ou la revendication 10, dans laquelle le traitement est administré par voie topique.

12. Méthode de contrôle de la croissance d'une bactérie, la méthode comprenant l'application, sur une zone ou surface non vivante qui est infectée ou suspectée d'être infectée ou capable d'être infectée par la bactérie, d'une formulation contenant (a) de l'acide usnique ou un usnate et (b) un sel métallique sélectionné parmi les sels de cuivre, les sels de bismuth et leurs mélanges, dans laquelle si le composant (a) est un usnate, les composants (a) et (b) ne sont pas le même composé.
